# EUROPEAN PATENT APPLICATION

(11) **EP 2 005 890 A2**
(43) Date of publication of application: **24.12.2008**
(21) Application number: 07740437.4
(22) Date of filing: 29.03.2007
(51) Int. Cl.: A61B 8/08

(54) **IMAGE PROCESSING DEVICE, ULTRASONIC IMAGING DEVICE USING THE SAME, AND IMAGE PROCESSING METHOD**

(30) Priority: 31.03.2006 JP 2006099123
(71) Applicant: National University Corporation Kyoto Institute of Technology, Sakyo-ku Kyoto-shi, Kyoto 606-8585 (JP)
(72) Inventor: FUKUZAWA, Masayuki, . (JP); YAMADA, Masayoshi, . (JP)
(74) Representative: Bosch, Matthias
(86) International application number: PCT/JP2007/056999
(87) International publication number: WO 2007/114305

(57) **Abstract**

An image processing device (8) includes: a frequency analysis section (12) for detecting an amplitude frame (PA) of a specific frequency component and a phase frame (PP) of the specific frequency component by performing a frequency analysis of time-series changes p_{x,y}(tᵢ) in echo intensity frame (p_{x,y}) or time-series changes q_{x,y}(tᵢ) in Doppler velocity frame (q_{x,y}) at various points within a cross-section of a specimen, the echo intensity frame (p_{x,y}) and the Doppler velocity frame (q_{x,y}) being each obtained by scanning the cross-section of the specimen with an ultrasonic beam; and an image generation section (13) for generating a two-dimensional image based on the amplitude of the specific frequency component and the phase of the specific frequency component thus detected. Thus, an image processing device can be provided which makes it possible to observe a two-dimensional shape of a specimen, the intensity of a periodic motion of the specimen, and spatial continuity in phase of the periodic motion of the specimen.

## Description

### TECHNICAL FIELD

The present invention relates to an image processing device that visualizes echo intensity, an ultrasonic imaging apparatus including the image processing device, and an image processing method.

### BACKGROUND ART

Conventionally, an ultrasonic imaging apparatus (ultrasonic diagnostic apparatus) has been used which visualizes the movement or function of *in vivo* tissue for observation by using an ultrasonic beam.

The visualization has been performed by using an echo signal obtained from an ultrasonic beam shined on and reflected by biomedical tissue serving as a specimen. Hitherto used examples of a method for visualizing an echo signal in such an ultrasonic imaging apparatus include: a B (Brightness) mode, in which a still image or moving image of a two-dimensional distribution of echo intensity is formed by electronic scanning with an ultrasonic beam; and an M (Motion) mode, in which a still image of time-series changes in echo intensity during a predetermined period of time is formed with an ultrasonic beam fixed.

As for major tissue such as heart tissue, liver tissue, and brain tissue, a tissue motion of interest to a doctor is related in large part to heartbeat or the pulsation of a blood flow. Therefore, it is useful to observe an intensity distribution of a periodic tissue motion and spatial continuity in phase of the periodic tissue motion.

The B-mode still image and the B-mode moving image have been used for direct observation of a two-dimensional shape of tissue and a tissue motion. However, because the B-mode moving image is updated from one display to another every moment, the periodicity of a tissue motion cannot be observed. Further, the B-mode still image and the B-mode moving image make it possible to observe a two-dimensional shape of tissue, but fail to make it possible to observe the intensity or phase of the cycle of a tissue motion.

On the other hand, the M-mode still image makes it possible to obtain a history of motions of tissue toward and away from an ultrasonic probe. As such, the M-mode still image is used for observation of the intensity and phase of a periodic tissue motion such as a pattern of motion of a cardiac valve. However, since the M-mode still image is not formed by scanning with an ultrasonic beam, the M-mode still image undesirably fails to make it possible to observe a two-dimensional shape of tissue. The M-mode still image makes it possible to observe the periodicity of a tissue motion, but fails to make it possible to observe how the intensity or phase of a periodic tissue motion is distributed within a specimen and what spatial continuity the phase has.

That is, the use of the B mode makes it possible to observe a two-dimensional shape, but undesirably fails to make it possible to observe the periodicity of a motion. On the other hand, the use of the M mode makes it possible to observe the periodicity of a motion, but undesirably fails to make it possible to observe a two-dimensional shape. Further, an ultrasonic diagnostic apparatus of Patent Document 1 detects the instantaneous velocity of tissue with use of a method called an ultrasonic Doppler method, thereby displaying the instantaneous velocity of tissue in an area of interest. However, since the ultrasonic diagnostic apparatus of Patent Document 1 is based on the instantaneous velocity of tissue, the ultrasonic diagnostic apparatus of Patent Document 1 fails to make it possible to observe the periodicity of a motion.

This has required a doctor to memorize a large number of B- and M-mode images in his/her head and build a relationship between a tissue shape and a tissue motion with use of his/her anatomical knowledge. Accordingly, there has been a demand for image processing that makes it possible to observe a two-dimensional shape of tissue and to observe the periodicity of a motion.

In order to observe the intensity of a periodic tissue motion with use of a two-dimensional image, the inventors proposes, in Non-patent Documents 1 and 2, a method for extracting the intensity of a specific frequency component of echo intensity by inter-frame image calculation of a B-mode moving image and visualizing the intensity. This makes it possible to observe the periodicity of a motion and a two-dimensional shape, thereby solving the foregoing problems with the B mode and the M mode.

Further, an ultrasonic diagnostic apparatus of Patent Document 2 is an apparatus that sequentially calculates velocities of a moving body within a specimen, corrects each of the velocities with use of a reference velocity, and displays data on the velocity thus corrected. This makes it possible to display a corrected velocity of a blood flow two-dimensionally or three-dimensionally and thereby display pulsation simply and effectively.

Further, an ultrasonic image processing apparatus of Patent Document 3 uses a tissue-tracking imaging method to generate, for each time phase, a velocity distribution image from which translational and rotational velocity components caused by body movement or the like have been removed, uses the velocity distribution image to perform a tracking process related to a predetermined site of tissue, and generates movement information image, thereby providing a more highly credible diagnostic image.

Further, an ultrasonic diagnostic apparatus of Patent Document 4 relates to generation of a 3D image with use of a 2D ultrasonic probe, and is intended to make it possible to observe the normal pulsations and/or irregular pulsations of a fast-moving human organ such as a heart on a three-dimensional moving image. Specifically, in solving a problem with inclusion of a plurality of systolic and diastolic periods of a heart within a scanning time when the heart is scanned with a 2D probe, the ultrasonic diagnostic apparatus of Patent Document 4 has means for specifying the phase of a cross-sectional surface with use of an electrocardiographic signal.

Further, an apparatus of Patent Document 5 for displaying a record of ultrasonic diagnostic images is an apparatus that makes it possible to easily grasp changes in medical condition. Specifically, the apparatus of Patent Document 5 switches displays between the present ultrasonic diagnostic images and the past ultrasonic diagnostic images, and the images are observed as moving images out of synchronization.

Furthermore, an invention of Patent Document 6 has as an object to provide an image forming method for generating an image indicative of a distribution of a periodic motion and an ultrasonic diagnostic apparatus capable of generating and displaying an image indicative of a distribution of a periodic motion. The invention of Patent Document 6 acquires time-series data from a plurality of sampling points distributed in space, calculates periodicity from the time-series data, and generates an image indicative of a relationship between the distribution of the sampling points and the periodicity.
Patent Document 1: Japanese Unexamined Patent Application Publication No. 84729/1996 (*Tokukaihei* 8-84729; published on April 2, 1996)
Patent Document 2: Japanese Unexamined Patent Application Publication No. 61958/2003 (*Tokukai* 2003-61958; published on March 4, 2003)
Patent Document 3: Japanese Unexamined Patent Application Publication No. 124636/2005 (*Tokukai* 2005-124636; published on May 19, 2005)
Patent Document 4: Japanese Unexamined Patent Application Publication No. 336255/2002 (*Tokukai* 2002-336255; published on November 26, 2002)
Patent Document 5: Japanese Unexamined Patent Application Publication No. 97451/1991 (*Tokukaihei* 3-97451; published on April 23, 1991)
Patent Document 6: Japanese Unexamined Patent Application Publication No. 173417/1996 (*Tokukaihei* 8-173417; published on July 9, 1996)
Non-patent Document 1: Jpn. J. Appl. Phys. Vol. 34, pp. 2854-2856 (published on March 18, 1995)
Non-patent Document 2: Jpn. J. Appl. Phys. Vol. 38, pp. 3385-3387 (published on February 5, 1999)

### DISCLOSURE OF THE INVENTION

In the case of use of the technique proposed in Patent Document 6, Non-patent Document 1, or Non-patent Document 2, the intensity of a specific frequency component of echo intensity can be calculated. However, there has been such a problem that a continuum of (continuity in) intensities of a specific frequency component cannot be found simply by calculating the intensities of the specific frequency component.

Further, each of Patent Documents 4 and 5 teaches means for identifying which heartbeat phase is indicated by a point in time where a tomographic image was taken entirely, but fails to teach means for extracting the location of a periodic motion within a cross-sectional surface, and thus having such a problem that the location cannot be extracted. Furthermore, each of Patent Documents 2 and 3 similarly fails to teach means for extracting the location of a periodic motion within a cross-sectional surface, thus having such a problem that the location cannot be extracted.

The present invention has been made in view of the foregoing problems. It is an object of the present invention to provide an image processing device and an image processing method that make it possible to observe a two-dimensional shape of a specimen, the intensity of a periodic motion of the specimen, and spatial continuity in phase of the periodic motion of the specimen.

In order to solve the foregoing problems, an image processing device of the present invention includes: frequency analysis means for detecting amplitude of a specific frequency component and a phase of the specific frequency component by performing a frequency analysis of time-series changes in echo intensity or in Doppler velocity at various points within a cross-section of a specimen, the echo intensity and the Doppler velocity being each obtained by scanning the cross-section of the specimen with an ultrasonic beam; and first image generation means for generating a two-dimensional image based on the amplitude of the specific frequency component and the phase of the specific frequency component thus detected.

Further, an image processing method of the present invention is an image processing method for use in an ultrasonic imaging apparatus, including the steps of: detecting amplitude of a specific frequency component and a phase of the specific frequency component by performing a frequency analysis of time-series changes in echo intensity or in Doppler velocity at various points within a cross-section of a specimen, the echo intensity and the Doppler velocity being each obtained by scanning the cross-section of the specimen with an ultrasonic beam; and generating a two-dimensional image based on the amplitude of the specific frequency component and the phase of the specific frequency component thus detected.

The image processing device and the image processing method can be used for an ultrasonic imaging apparatus. Moreover, the image processing device obtains time-series changes in echo intensity or in Doppler velocity from outside of the image processing device. The echo intensity can be obtained by scanning a cross-section of a specimen with an ultrasonic beam. In particular, the present invention detects not only the amplitude of a specific frequency component but also the phase of the specific frequency component by performing a frequency analysis of the time-series changes in echo intensity or in Doppler velocity. The present invention can calculate an intensity distribution of a periodic tissue motion of a specimen by detecting the amplitude of a specific frequency component and generating a two-dimensional image based on the amplitude of the specific frequency component. Furthermore, in particular, since the present invention detects the phase of the specific frequency component, the present invention can generate an image with continuity in intensity of the specific frequency component by generating a two-dimensional image in accordance with the phase. This makes it possible to obtain a two-dimensional image with spatial continuity in phase.

This makes it possible to observe the periodicity of a tissue motion, a two-dimensional shape of tissue, and spatial continuity in phase.

Additional objects, features, and strengths of the present invention will be made clear by the description below. Further, the advantages of the present invention will be evident from the following explanation in reference to the drawings.

### BRIEF DESCRIPTION OF THE DRAWINGS

Fig. 1 is a block diagram showing an arrangement of a main part of an image processing section according to an embodiment of the present invention.
Fig. 2(a) shows a pattern diagram of a B-mode image and a graph showing a relationship between luminance values at various points on the image and time.
Fig. 2(b) is a graph obtained by performing a frequency analysis for amplitude calculation on the graph of Fig. 2(a).
Fig. 2(c) is a graph obtained by performing a frequency analysis for phase calculation on the graph of Fig. 2(a).
Fig. 3 is a block diagram schematically showing an arrangement of an ultrasonic imaging apparatus according to an embodiment of the present invention.
Fig. 4 is a block diagram showing a modified example of the ultrasonic imaging apparatus of Fig. 3 according to an embodiment of the present invention.
Fig. 5 is a block diagram showing an arrangement of a main part of an image processing section according to another embodiment of the present invention.

### REFERENCE NUMERALS

5 Ultrasonic wave transmitting and receiving section (ultrasonic wave transmitting and receiving means)
8 Image processing section (image processing device)
12 Frequency analysis section (frequency analysis means)
13 Image generation section (first image generation means)
23 Fourier transform section (Fourier transform means)
25 Amplitude LUT (amplitude look-up table)
26 Phase LUT (phase look-up table)
36 Image generation section (first image generation means)
37 Two-dimensional image LUT (two-dimensional image look-up table)
41 Profile image generation section (second image generation means)
43 Profile LUT (second two-dimensional image look-up table)
45 Continuous phase curve detection section (detection means)
p_{x,y} Echo intensity frame
q_{x,y} Doppler velocity frame
p_{x,y}(tᵢ) Time history of echo intensity frames (time-series changes in echo intensity frame)
v_{x,y}(tᵢ) Instantaneous optical flow velocity (time-series changes in echo intensity frame)
PA Amplitude frame of a specific frequency component
PP Phase frame of a specific frequency component
t_{HB} Heartbeat cycle

### BEST MODE FOR CARRYING OUT THE INVENTION

### [Embodiment 1]

An embodiment of the present invention will be described below with reference to the drawings.

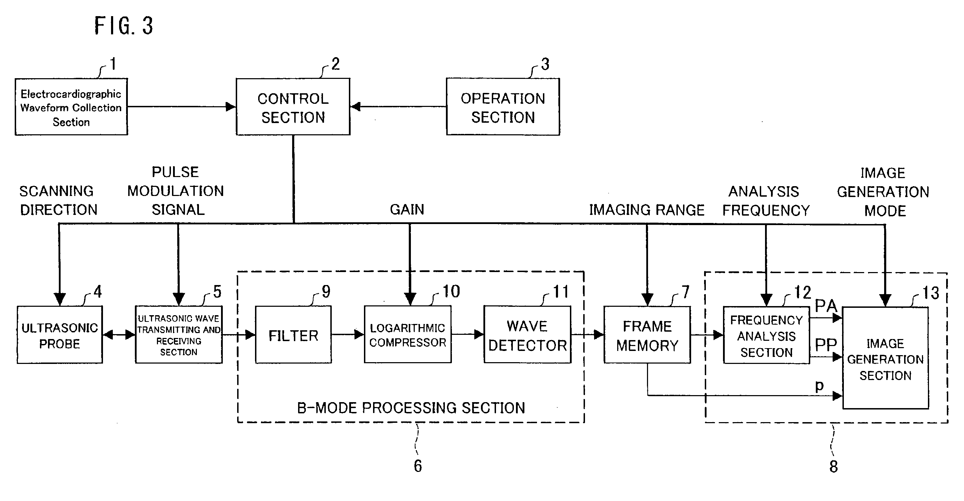

Fig. 3 is a block diagram schematically showing an arrangement of an ultrasonic imaging apparatus of the present invention. The ultrasonic imaging apparatus, used mainly in a medical field, enables detailed observation of the condition of a fetus or the like with use of an ultrasonic echo.

As shown in Fig. 3, the ultrasonic imaging apparatus of the present invention includes an electrocardiographic waveform collection section 1, a control section 2, an operation section 3, an ultrasonic probe 4, an ultrasonic wave transmitting and receiving section (ultrasonic wave transmitting and receiving means) 5, a B-mode processing section (B-mode processing means) 6, a frame memory 7, and an image processing section (image processing device) 8. Furthermore, as shown in Fig. 3, the B-mode processing section 6 includes a filter 9, a logarithmic compressor 10, and a wave detector 11. Further, as shown in Fig. 3, the image processing section 8 includes a frequency analysis section (frequency analysis means) 12 and an image generation section (two-dimensional image generating section; first image generation section) 13.

The operation section 3 is an interface via which a user such as a doctor switches between image modes. Although not shown, the image processing section 8 is followed by a display section such as a liquid crystal display.

The electrocardiographic waveform collection section 1 collects electrocardiographic waveforms from outside, determines (calculates) a heartbeat cycle and a time phase from the electrocardiographic waveforms, and then sends the heartbeat cycle and the time phase to the control section 2. The control section 2 mainly sends, to the frequency analysis section 12, the heartbeat cycle and the time phase or an analysis frequency and an initial phase that have been manually designated via the operation section 3. The image processing section 8 can replace an image processing section on an existing ultrasonic imaging apparatus, and can be incorporated as a new feature into an existing ultrasonic imaging apparatus. It should be noted that the ultrasonic imaging apparatus of the present invention encompasses an ultrasonic imaging apparatus including an image processing section 8 in advance.

Furthermore, the control section 2 sends a scanning-direction indicator signal to the ultrasonic probe 4, sends a pulse modulation signal to the ultrasonic wave transmitting and receiving section 5, sends a gain to the logarithmic compressor 10, and sends an image-generation-mode designating signal to the image generation section 13.

The control section 2 determines the gain on the basis of a value designated by the user via the operation section 3, and generates the image-generation-mode designating signal in accordance with a choice made by the user via the operation section 3.

The ultrasonic probe 4 includes a plurality of transducer arrays (not shown) disposed so as to emit ultrasonic waves (ultrasonic beams) in different directions, e.g., in directions extending radially in the form of a circular sector. Each transducer array transmits an ultrasonic wave to (tissue of) a cross-section of a specimen, and receives an ultrasonic wave from the scanning direction in which the ultrasonic wave has been transmitted. Each transducer array switches between ON and OFF electronically in accordance with the scanning-direction indicator signal sent from the control section 2. This makes it possible to sequentially switch between directions in which the ultrasonic probe 4 transmits and receives ultrasonic waves. It should be noted that each transducer array is fixed to the ultrasonic probe 4 and that each transducer array transmits and receives ultrasonic waves in a predetermined direction. Therefore, a scanning direction in which ultrasonic waves are transmitted and received is uniquely determined once a transducer array is determined.

The ultrasonic wave transmitting and receiving section 5 serves to generate, by pulse modulation based on the pulse modulation signal sent from the control section 2, an ultrasonic wave (transmission ultrasonic wave) that a transducer array of the ultrasonic probe 4 transmits to the cross-section of the specimen, and to amplify an ultrasonic wave (reception ultrasonic wave) that the transducer array has received from the specimen.

As shown in Fig. 3, the B-mode processing section 6 is disposed behind the ultrasonic wave transmitting and receiving section 5, and includes the filter 9, the logarithmic compressor 10, and the wave detector 11, which are provided in this order starting from the ultrasonic wave transmitting and receiving section 5. The B-mode processing section 6 serves to express, as a B-mode signal I(d) that is indicated by a function of the distance d from the ultrasonic probe 4 to the cross-section of the specimen, echo intensity at each point within the cross-section of the specimen in each scanning direction of the ultrasonic probe 4. The B-mode signal I(d) is obtained by indicating the echo intensity by a function of the distance d. As such, the B-mode signal I(d) is referred to also as "echo intensity signal".

The filter 9 receives an ultrasonic wave from the ultrasonic wave transmitting and receiving section 5, selects only an echo signal from the ultrasonic wave, and transmits the echo signal. The logarithmic compressor 10 performs logarithmic compression on the echo signal transmitted through the filter 9. In so doing, the logarithmic compressor 10 uses the gain inputted from the control section 2. The wave detector 11 serves to detect a pulse-modulated component of the ultrasonic wave finished with the logarithmic compression, to generate a B-mode signal I(d), and to output the B-mode signal I(d).

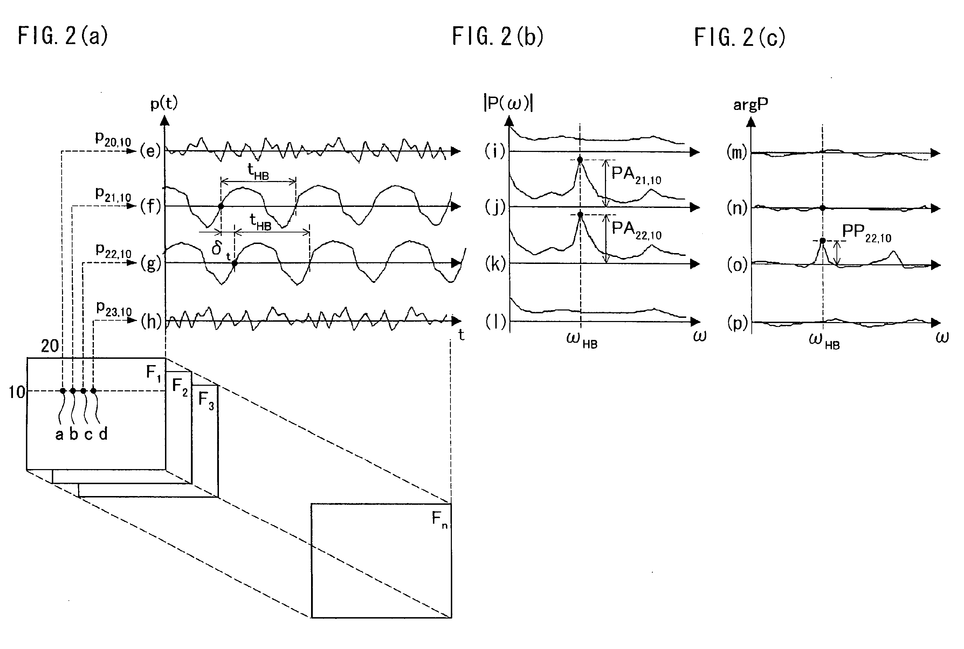

The frame memory 7 determines echo intensity frame p_{x,y} at each point (x,y) within the cross-section of the specimen by using a plurality of B-mode signals I(d) sent from the B-mode processing section 6 for each of the different scanning directions of ultrasonic waves, generates a succession of B-mode images Fi (i = 1, 2, ..., n) serving as two-dimensional distribution images of the echo intensity frame p_{x,y}, and updates the B-mode images Fi (see Fig. 2(a)). It should be noted here that i is a variable that indicates the order in which the two-dimensional distribution images are generated. As described above, each transducer array has a predetermined scanning direction, each point (x,y) within the cross-section of the specimen is uniquely determined once a transducer array and the distance d from the ultrasonic probe 4 to the specimen are determined. Further, the frame memory 7 receives an imaging range signal from the control section 2. It should be noted here that the imaging range signal expresses a range of signals to be contained as B-mode images in a circular sector from among the echo intensity signals (B-mode signals I(d)).

That is, the range within which the ultrasonic probe 4 can transmits and receives ultrasonic waves, i.e., the range of d in I(d) does not necessarily correspond to the radius R of a circular sector to be generated as a B mode image, so that it is possible that r < d.

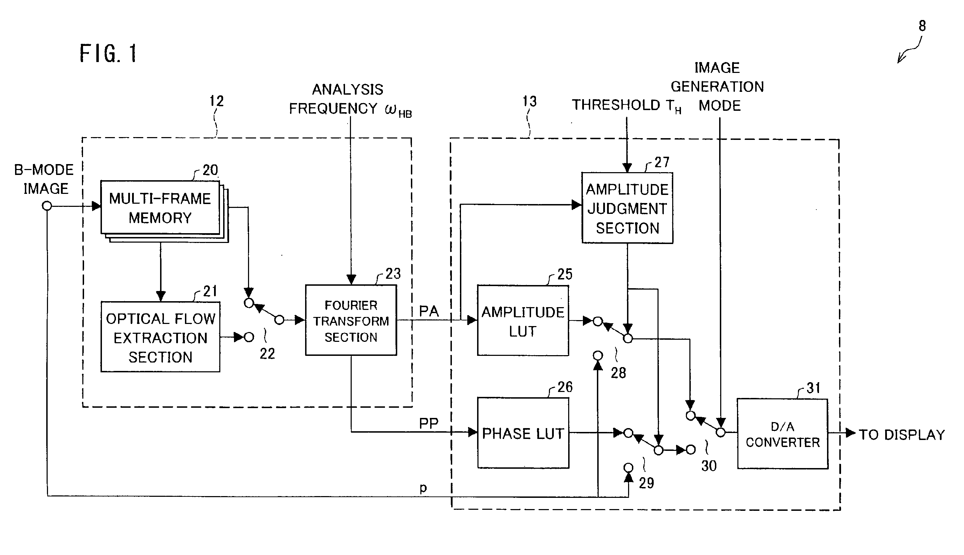

The following explains the image processing section 8, which is the most important part of the present invention. As shown in Fig. 1, the image processing section 8 includes the frequency analysis section 12 and the image generation section 13. As shown in Fig. 1, the frequency analysis section 12 includes a plurality of memories (such a plurality of memories being referred to also as "multi-frame memory") 20, an optical flow extraction section 21, a first selection mechanism (i.e., a mechanism that sends either of two types of signal to the subsequent stage in accordance with a judgment signal inputted from outside) 22, and a Fourier transform section 23. The frequency analysis section 12 receives all or part of the B-mode images F1 to Fi generated one after another by the frame memory 7, calculates time-series changes (p_{x,y}(tᵢ) in or the instantaneous optical flow velocity v_{x,y}(tᵢ) of) echo intensity frame p_{x,y} therefrom, performs an frequency analysis of the time-series changes, calculates an amplitude frame PA (Periodical Amplitude)_{x,y} and phase frame PP (Periodical Phase)_{x,y} of a specific frequency component designated by the control section 2, and outputs the amplitude frame PA_{x,y} and the phase frame PP_{x,y} to the image generation section 13.

The multi-frame memory 20 accumulates, in chronological order, all or part of the B-mode images F1 to Fi sent from the frame memory 7, and generates a time history p_{x,y}(tᵢ) of echo intensity frames p_{x,y} within a given amount of time.

The optical flow extraction section 21 receives the time history p_{x,y}(tᵢ) of echo intensity frames p_{x,y} from the multi-frame memory 20, and extracts the instantaneous optical flow velocity v_{x,y}(tᵢ) from the time history p_{x,y}(tᵢ) of echo intensity frames p_{x,y}, for example, by using a gradient method.

The first selection mechanism 22 serves to choose either the multi-frame memory 20 or the optical flow extraction section 21 to be connected to the Fourier transform section 23.

In cases where there are only small variations in the contrast of the echo intensity frames p_{x,y} among specimens, the first selection mechanism 22 connects the multi-frame memory 20 to the Fourier transform section 23. That is, the time history p_{x,y}(tᵢ) of echo intensity generated by the multi-frame memory 20 is inputted to the Fourier transform section 23. On the other hand, in cases where there are large variations in the contrast of the echo intensity frames p_{x,y} among the specimens, the first selection mechanism 22 connects the optical flow extraction section 21 to the Fourier transform section 23. That is, the instantaneous optical flow velocity v_{x,y}(tᵢ) generated by the optical flow extraction section 21 is inputted to the Fourier transform section 23.

The Fourier transform section 23 receives the time history p_{x,y}(t_{¡}) of echo intensity or the instantaneous optical flow velocity v_{x,y}(tᵢ), performs Fourier transform thereon, and detects the amplitude of the specific frequency component and the phase of the specific frequency component. Further, the optical flow velocity v_{x,y}(tᵢ) can be calculated with use of time-series changes in echo intensity at points (x,y) and time-series changes in echo intensity near the points (x,y), for example, according to a gradient method.

Further, as shown in Fig. 1, the amplitude PA of the specific frequency component and the phase PP of the specific frequency component are detected, for example, by extracting, from the Fourier transform of the time history of echo intensity, a frequency component corresponding to the analysis frequency ω_{HB} sent from the control section 2 to the Fourier transform section 23 of the frequency analysis section 12.

The following explains the operation of the frequency analysis section 12 with reference to Figs. 2(a) through 2(c). For convenience of explanation, the following explanation assumes that the time history p_{x,y}(tᵢ) of echo intensity is used. However, the same applies to a case where the optical flow velocity v_{x,y}(tᵢ) is used.

As shown in Fig. 2(a), the multi-frame memory 20 accumulates n B-mode images F1 to Fn. Furthermore, the multi-frame memory 20 calculates the time history p_{x,y}(tᵢ) of echo intensity at the coordinates (x,y) of these B-mode images. It should be noted here that the term "coordinates (x,y)" corresponds to the term "various points within a cross-section of a specimen" recited in the claims.

As shown in Fig. 2(a), the present example shows a relationship between luminance p(t) and time (t) at a point a (20,10), a point b (21, 10), a point c (22, 10), and a point d (23, 10). The points a, b, c, and d correspond to waveforms (e), (f), (g), and (h), respectively.

As shown in Fig. 2(a), the waveforms (e) and (h) are noise-like waveforms. This shows that there is random motion at the points (a) and (d). On the other hand, each of the waveforms (f) and (g) has a certain level of periodicity. As shown in Fig. 2(b), a single cycle of each of the waveforms (f) and (g) is denoted by t_{HB} (heartbeat), and is substantially identical to the cycle of a single heartbeat. The respective cycles of the waveforms (f) and (g) are both denoted identically by t_{HB}, but the waveforms (f) and (g) differ by δt minutes in terms of time at which p(t) is 0.

Next, the Fourier transform section 23 performs a frequency analysis on each of the waveforms (e) to (h) in order to detect the amplitude of a specific frequency component. The results are shown in Fig. 2(b). Fig. 2(b) shows waveforms (i) to (I) respectively obtained by performing frequency analyses on the waveforms (e) to (h). In Fig. 2(b), the vertical axis shows frequency component |P(ω)|, and the horizontal axis shows angular frequency ω.

As shown in Fig. 2(b), the waveforms (i) and (I), respectively obtained by performing frequency analyses on the waveforms (e) and (h), are waveforms substantially identical in frequency component size to each other, each without any peak where the frequency component is prominent. On the other hand, each of the waveforms (j) and (k), obtained by performing frequency analyses on the constantly periodic waveforms (f) and (g), has a peak (where the frequency component is prominent) at an angular frequency of ω_{HB}. It should be noted here that the values of the peak frequency components are the amplitude frame PA_{21,10}, and the amplitude frame PA_{22,10}. Further, ω_{HB} can be appropriately determined by a doctor using the ultrasonic imaging apparatus. Further, ω_{HB} may be determined in synchronization with an electrocardiogram.

Furthermore, the Fourier transform section 23 performs a frequency analysis on each of the waveforms (e) to (h) in order to detect the phase of a specific frequency component. The results are shown in Fig. 2(c). Fig. 2(c) shows waveforms (m) to (p) respectively obtained by performing frequency analyses on the waveforms (e) to (h) and detecting phases with reference to the phase of the waveform (f). In Fig. 2(c), the vertical axis shows phase argP, and the horizontal axis shows angular frequency ω. As shown in Fig. 2(c), each of the waveforms (m) and (p), which respectively correspond to the non-periodic noise-like waveforms (e) and (h), has an indefinite value in phase at ω_{HB}. However, the waveform (n), which corresponds to the waveform (f), has a value of 0 in phase at ω_{HB}. Meanwhile, the waveform (o), which corresponds to the waveform (g), has a nonzero value PP_{22,10} in phase at ω_{HB}.

As shown in Figs. 2(a) and 2(b), such a nonzero value PP_{22,10} is obtained because the waveforms (f) and (g) exhibit constant periodicity in terms of the luminance P(t) and differ in phase by δt. It should be noted that although the nonzero value PP_{22,10} varies periodically within a range of ±180 degrees, the nonzero value PP_{22,10} increases in proportion to the value of δt as long as δt is not more than (within a range of) 1/2 of t_{HB}.

As shown in Fig. 1, the image processing section 13 includes an amplitude LUT (look-up table) 25, a phase LUT 26, an amplitude judgment section 27, a second selection mechanism 28, a third selection mechanism 29, a fourth selection mechanism 30, and a D/A converter 31.

Moreover, the image processing section 13 receives an echo intensity frame p from the frame memory 7, receives an amplitude frame PA and phase frame PP of a specific frequency component from the frequency analysis section 12, and generates a grayscale gradation image or a color gradation image by using, as an evaluated value, any one of those values or a result of calculation performed based on those values. The image thus generated is sent to the subsequent display. More specifically, the amplitude frame PA is inputted to the amplitude LUT 25 and the amplitude judgment section 27. The phase frame PP is inputted to the phase LUT 26. The echo intensity frame p is inputted to the second selection mechanism 28 and the third selection mechanism 29.

The amplitude LUT 25 contains a color gradation value corresponding to the value of the amplitude frame PA. In accordance with this color gradation value, the control section 2 converts the value of the amplitude frame PA into a color gradation value. Meanwhile, the phase LUT 26 contains a color gradation value corresponding to the value of the phase frame PP. In accordance with this color gradation value, the control section 2 converts the value of the phase frame PP into a color gradation value. It should be noted that examples of the amplitude LUT 25 and the phase LUT 26 include, but are not limited to, RAMs, ROMs, and EEPROMs, provided a color gradation value corresponding to the value of the amplitude frame PA or the phase frame PP can be stored.

The amplitude judgment section 27 receives a threshold T_{H} from the control section 2, makes a comparison between the threshold T_{H} and the amplitude (amplitude frame) PA, sends the second selection mechanism 28 and the third selection mechanism 29 a switching signal corresponding to the magnitude relation between the threshold T_{H} and the amplitude frame PA, and switches, for each pixel on the frame, signals to be sent to the stage subsequent to the selection mechanisms 28 and 29. More specifically, in cases where the amplitude frame PA is less than the threshold T_{H}, the echo intensity frame p is sent to the display (subsequent stage) via the second selection mechanism 28 and the third selection mechanism 29, and the amplitude frame PA and the phase frame PP are blocked. On the other hand, in cases where the amplitude frame PA is not less than the threshold T_{H}, the amplitude frame PA and the phase frame PP are sent via the second selection mechanism 28 and the third selection mechanism 29 to the fourth selection mechanism 30 located closer to the display (subsequent stage), and the echo intensity frame p is blocked. It should be noted that there is a time where the echo intensity frame p is higher in frame rate than the amplitude frame PA and the phase frame PP. That is, the echo intensity frame p, the amplitude frame PA, and the phase frame PP are not necessarily identical in frame rate to one another, and may be different in frame rate from one another.

The second selection mechanism 28 is disposed behind the amplitude LUT 25. In accordance with a switching signal sent from the amplitude judgment section 27, the second selection mechanism 28 chooses either the amplitude frame PA or the echo intensity frame p to be sent toward the display from the second selection mechanism 28. Meanwhile, the third selection mechanism 29 is disposed behind the phase LUT 26. In accordance with a switching signal sent from the amplitude judgment section 27, the third selection mechanism 29 chooses either the phase frame PP or the echo intensity frame p to be sent toward the display from the third selection mechanism 29.

The fourth selection mechanism 30 is disposed behind the second selection mechanism 28 and the third selection mechanism 29. Moreover, the fourth selection mechanism 30 chooses either an output from the second selection mechanism 28 (the value of an echo intensity frame p or an amplitude frame PA at each point (x,y)) or an output from the third selection mechanism 29 (the value of an echo intensity frame p or a phase frame PP at each point (x,y)) to be outputted toward the display. This switching is performed based on an image generation mode signal inputted from the control section 2 to the fourth selection mechanism 30.
Furthermore, the D/A converter 31, disposed behind the fourth selection mechanism 30, converts digital data into analog data, and then outputs the analog data toward the display.

Incidentally, the B-mode processing section 6 of Fig. 3 generates B-mode signals I(d) one after another, thereby updating the frame memory 7 so that the frame memory 7 always has the most recent echo intensity frame p. The multi-frame memory 20 discards the earliest echo intensity frame p and accumulates data on the most recent echo intensity frame p every time the frame memory 7 is updated, thereby always retaining the most recent n echo intensity frames p.

The frequency analysis section 12 generates an amplitude frame PA and phase frame PP of a specific frequency component from *m* (m ≤ n) echo intensity frames, contained in the multi-frame memory 20, which are most recent as of the start of an analysis. It should be noted here that m is the number of frames that are analyzed by the frequency analysis section 12.

If the number n of echo intensity frames p that are retained in the multi-frame memory 20 is made sufficiently larger than the number m of frames that are analyzed by the frequency analysis section 12, the generation of a pair of an amplitude frame PA and a phase frame PP does not necessarily require the same amount of time as an update of the frame memory 7.

The image generation section 13 regenerates a display frame every time an echo intensity frame p, an amplitude frame PA of a specific frequency component, or a phase frame PP of the specific frequency component is updated and repeatedly displays the display frame, thereby making it possible to provide a moving image to a doctor using the ultrasonic imaging apparatus.

Thus, in an image that is shown by a display such as a liquid crystal display, a pixel where the amplitude of a specific frequency component (i.e., the intensity of a periodic tissue motion) is less than the threshold T_{H} has the monochrome gradation value of echo intensity p. That is, the image is a B-mode image. On the other hand, a pixel where the amplitude of a specific frequency component (i.e., the intensity of a periodic tissue motion) is not less than the threshold T_{H} has the color gradation value of a phase frame PP. Therefore, only an area indicative of a certain or higher level of intensity of a periodic tissue motion is colored, so that a phase relation between tissues is expressed as gradations of color. Therefore, the spatial continuity in phase, as well as the intensity of a periodic tissue motion, can be expressed as a two-dimensional image.

In particular, the present invention performs color coding by calculating a phase frame PP of a specific frequency component, thus bringing about an effect of enabling observation of the continuity in (continuum of) blood-flowing tissue such as pulsatile tissue.

In the foregoing, the amplitude of a specific frequency component and the phase of the specific frequency component are obtained through Fourier transform. However, this is merely an example. The amplitude of a specific frequency component and the phase of the specific frequency component may be obtained, for example, through an autoregressive model.

Further, examples of specific frequency components include, but are not limited to, frequency components equivalent to a heartbeat cycle, and encompass frequency components such as second and third harmonic components respectively obtained by multiplying the heartbeat cycle by integers of 2 and 3. That is, examples of the specific frequency component encompass frequency components other than the heartbeat cycle.

Further, the image processing device and the ultrasonic imaging apparatus of the present invention can be suitably used in particular for pulsatile tissue (i.e., tissue that moves periodically) such as the head of a newborn baby.

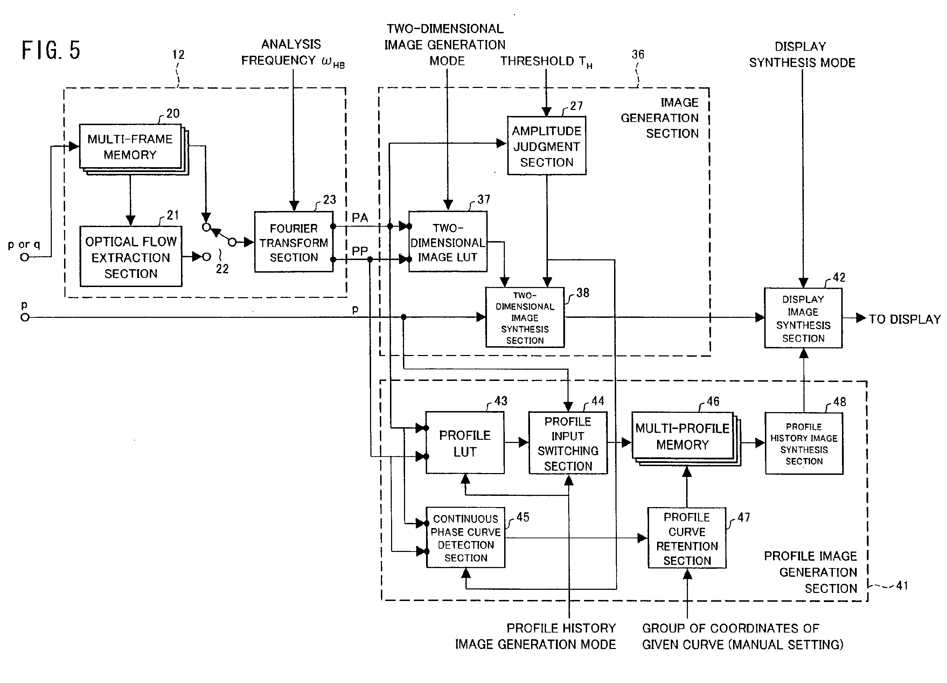

Further, the image generation section 13 shown in Fig. 1 may be replaced by such an image generation section (first image generation means) 36 as shown in Fig. 5. As shown in Fig. 5, the image generation section 36 includes a two-dimensional image LUT (first two-dimensional image look-up table) 37, an amplitude judgment section 27, and a two-dimensional image synthesis section 38.

The two-dimensional image LUT 37 receives an amplitude frame PA and a phase frame PP from the frequency analysis section 12, and receives a signal indicative of a two-dimensional image generation mode from the control section 2 (see Fig. 3).

The two-dimensional image LUT 37 contains color gradations values respectively corresponding to the value of the amplitude frame PA, the value of the phase frame PP, and a combination thereof, and these color gradation values are rewritten in accordance with the two-dimensional image generation mode. Possible examples of the color gradation values obtained from the combination of the value of the amplitude frame PA and the value of the phase frame PP include color gradation values that are obtained by expressing the amplitude by brightness of color and the phase by a hue.

The two-dimensional image synthesis section 38 receives an echo intensity frame p from the frequency analysis section 12, and receives a switching signal from the amplitude judgment section 27. Furthermore, in accordance with the switching signal, the two-dimensional image synthesis section 38 extracts such a gradation value from the two-dimensional image LUT 37.

Moreover, in accordance with the switching signal, the two-dimensional image synthesis section 38 chooses either the echo intensity frame p or the color gradation value for each pixel of a two-dimensional image, synthesizes the two-dimensional image, and outputs the two-dimensional image. The two-dimensional image synthesis section 38 re-synthesizes the two-dimensional image every time the amplitude frame PA, the phase frame PP, or the echo intensity frame p is updated. It should be noted that the amplitude frame PA, the phase frame PP, and the echo intensity frame p are not necessarily updated with identical cycles.

### [Embodiment 2]

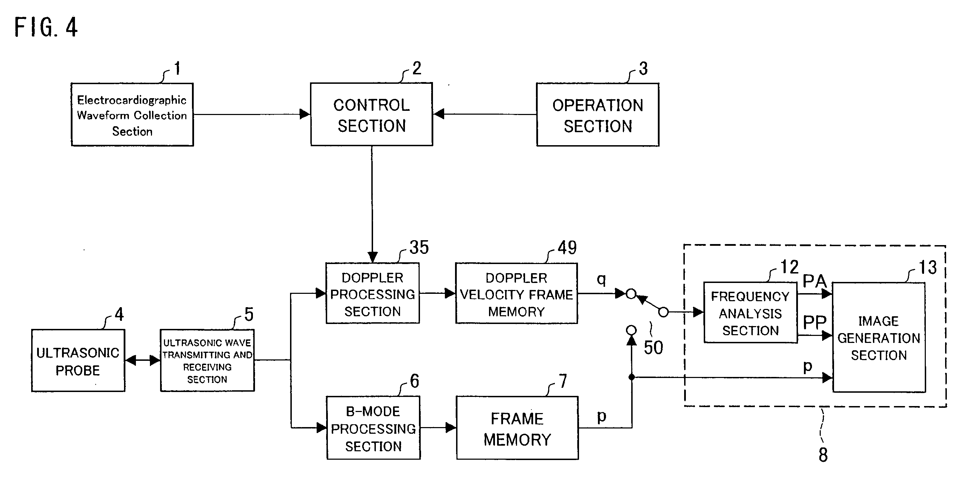

Furthermore, an ultrasonic imaging apparatus according to another embodiment of the present invention will be described below with reference to Fig. 4. The following describes only differences between the present embodiment and the above embodiment and omits descriptions of arrangements and functions identical to those of the above embodiment.

In addition to the arrangement of Embodiment 1 shown above in Fig. 3, the ultrasonic imaging apparatus of the present embodiment includes a Doppler processing section 35, a Doppler velocity frame memory 49, and a selection mechanism 50. Specifically, as shown in Fig. 4, an output from the ultrasonic wave transmitting and receiving section 5 is inputted not only to the B-mode processing section 6 but also to the Doppler processing section 35, and the Doppler processing section 35 is followed by the Doppler velocity frame memory 49. That is, the Doppler processing section 35 and the Doppler velocity frame memory 49 are provided in parallel with the B-mode processing section 6 and the frame memory 7.

The selection mechanism 50 is disposed behind the Doppler velocity frame memory 49 and in front of the frequency analysis section 12, and inputs either an output signal of the frame memory 7 or an output signal of the Doppler velocity frame memory 49 to the frequency analysis section 12. That is, the selection mechanism 50 can choose either the frame memory 7 or the Doppler velocity frame memory 49 to be connected to the frequency analysis section 12.

The Doppler processing section 35 detects a Doppler shift component from an ultrasonic echo signal sent from the ultrasonic wave transmitting and receiving section 5, thereby generating a Doppler velocity signal vd(d) corresponding to the moving velocity of tissue within a specimen.

By using the Doppler velocity signal vd(d) sent from the Doppler processing section, the Doppler velocity frame memory 49 determines a Doppler velocity frame q_{x,y} at each point (x,y) within a cross-section of the specimen. In cases where the specimen's tissue is so uneven that the tissue motion cannot be easily reflected in the contrast of echo intensity, the fifth selection mechanism 50 is controlled by the control section 2 so as to connect the Doppler velocity frame memory 49 to the frequency analysis section 12. In cases where the fifth selection mechanism 50 connects the frame memory 7 to the frequency analysis section 12, there is no difference between Embodiments 1 and 2. Therefore, such a case will not be described.

The image processing section 8 is arranged in the same manner as in Embodiment 1, and differs from Embodiment 1 only in that a Doppler velocity frame q_{x,y} is newly added as an input into the frequency analysis section 12.

Whereas an echo intensity frame p_{x,y} reflects the reflectance of an ultrasonic wave by a specimen, a Doppler velocity frame q_{x,y} reflects the moving velocity of the specimen's tissue. Therefore, an output obtained from the frequency analysis section 12 in cases where the Doppler velocity frame memory 49 is connected to the frequency analysis section 12 is based on a periodic change in the moving velocity of the specimen's tissue. Therefore, the present embodiment also makes it possible to observe a two-dimensional shape of a specimen, the intensity of a periodic motion of the specimen, and spatial continuity in phase of the motion of the specimen.

It should be noted that the fifth selection mechanism 50 is not an arrangement indispensable to the present embodiment. That is, the fifth selection mechanism 50 may be removed so that the Doppler velocity frame memory 49 is connected to the frequency analysis section 12 without connecting the frame memory 7 to the frequency analysis section 12. That is, the present embodiment may be arranged so as to input only a Doppler velocity frame q_{x,y} to the frequency analysis section 12.

### [Embodiment 3]

Furthermore, an ultrasonic imaging apparatus according to another embodiment of the present invention will be described below with reference to Fig. 5. The following describes only differences between the present embodiment and the above embodiments and omits descriptions of arrangements and functions identical to those of the above embodiments.

In addition to the arrangements of Embodiments 1 and 2, as shown in Fig. 5, the ultrasonic imaging apparatus of the present embodiment includes a profile image generation section (second image generation section) 41 and a display image synthesis section 42. The profile image generation section 41 is disposed behind the frequency analysis section 12 so as to be in parallel with the image generation section 36, and the display image synthesis section 42 is disposed behind the image generation section 36 and the profile image generation section 41.

The image generation section 36 may be replaced by the image generation section 13. The frequency analysis section 12 and the image generation section 36 are identical in content to those already described, and therefore will not be described below.

The profile image generation section 41 includes a profile LUT (second two-dimensional image look-up table) 43, a profile input switching section (input switching section) 44, a continuous phase curve detection section (curve detection section; detection means) 45, a multi-profile memory (profile memory) 46, a profile curve retention section (curve retention section) 47, and a profile history image synthesis section (history image synthesis section) 48.

The profile LUT 43 receives an amplitude frame PA and a phase frame PP from the frequency analysis section 12, and receives a profile history image generation mode signal from the control section 2 (see Fig. 3).

As with the two-dimensional image LUT 37 described above, the profile LUT 43 contains color gradation values respectively corresponding to the value of the amplitude frame PA, the value of the phase frame PP, and a combination thereof, and has the same functions as the two-dimensional image LUT 37. However, as shown in Fig. 5, the profile LUT 43 is disposed independently of the two-dimensional image LUT 37. The color gradation values are rewritten in accordance with the profile history image generation mode signal, which differs from a two-dimensional image generation mode signal.

The input switching section 44 receives an echo intensity frame p from the frame memory 7, receives the profile history image generation mode signal from the control section 2, and receives a color gradation value from the profile LUT 43. Moreover, in accordance with the profile history image generation mode signal, the input switching section 44 chooses either the echo intensity frame p or a frame based on the color gradation value to be sent to the subsequent multi-profile memory 46.

The curve detection section 45 receives the amplitude frame PA and the phase frame PP, and automatically extracts (a group of coordinates of) a continuous phase curve from the amplitude frame PA and the phase frame PP. It should be noted that it may be useful to detect, in addition to phase continuity, an area where amplitude is at a certain level or higher. In this case, as shown in Fig. 5, the curve detection section 45 may receive a switching signal from the amplitude judgment section 27 instead of receiving the amplitude frame PA.

The curve retention section 47 retains (the group of coordinates of) the phase continuous curve sent from the curve detection section 45 or (a group of coordinates of) a curve manually designated by an operator, and notifies the profile memory 46 of the curve.

It should be noted that the operator designates a curve in accordance with an area of diagnostic interest. For example, in cases where a child with a brain hemorrhage has a blood clot formed near his/her artery, the operator can designate a curve, for example, by drawing the curve around the blood clot on a B-mode image and observing a motion along the curve. It should be noted that the operator can actually designate a curve, for example, by drawing the curve on a B-mode image with a touch pen.

The profile memory 46 accumulates a predetermined number of frames sent from the profile input switching section 44, as reckoned from the present, and retains, for these frames, a group of pixel values corresponding to (the group of coordinates of) the curve of which the curve retention section 47 has notified the profile memory 46.

The history image synthesis section 48 synthesizes a two-dimensional image (profile history image) from a time history of the group of pixel values of the curve stored in the profile memory 46, and then outputs the two-dimensional image. For example, the history image synthesis section 48 extracts pixel values along a profile curve and uses the pixel values as a single vertical line of an image.

When such "single vertical lines" at the respective points of time in a history are arranged in a direction from right to left as reckoned from the present to the past, such an image can be generated that the vertical axis shows space coordinates along a profile line and the horizontal axis shows time elapsed.

In a special case of the image (output image) thus generated, i.e., in cases where a straight line extending along the direction of an ultrasonic beam is chosen as a curve and a monochrome gradation of echo intensity p is chosen as a profile history image generation mode, the history image synthesis section 48 produces an output equivalent to a so-called M-mode image.

The history image synthesis section 48 re-synthesizes only a single vertical line of the output image every time the amplitude frame PA, the phase frame PP, or the echo intensity frame p is updated.

The display image synthesis section 42 receives the two-dimensional image from the image generation section 36, and receives the profile history image from the profile image generation section 41. Moreover, in accordance with a display synthesis mode signal sent from the control section 2, the display image synthesis section 42 outputs either of the images to the display.

That is, the foregoing arrangement does not extract a motion from an M-mode image (M-mode echo image), but extracts a first-dimensional profile from among the periodicity (two-dimensional distribution) of a motion extracted from a B-mode moving image, draws the first-dimensional profile as a single vertical line, draws such single vertical lines from the left to the right as time elapses, and repeats the process, thereby visualizing a history of the periodicity and phase of the motion.

Further, in the M mode, a first-dimensional profile is limited to the direction in which an ultrasonic wave is emitted. However, in the present embodiment, how a first-dimensional profile is chosen and how an ultrasonic beam is chosen are not limited by the direction in which an ultrasonic beam is emitted. Further, as described above, a curve to be visualized is either calculated by automatically extracting (a group of coordinates of) a continuous phase curve by the curve detection section 45, or manually designated externally by an operator.

Further, as described above, the input switching section 44 sends, to the profile memory 46, an echo intensity frame p, the value of an amplitude frame PA of a specific frequency component, the value of a phase frame PP, or a frame based on a color gradation value corresponding to a combination of these values.

First, a case where the value of an amplitude frame PA of a specific frequency component, the value of a phase frame PP, or a frame based on a color gradation value corresponding to a combination to these values is sent to the profile memory 46 is explained.

In this case, for example, if a doctor who observes a blood flow obstruction around a blood clot of ventricular hemorrhage draws a curve in an area of interest around the blood clot and a history of a tissue motion on the curve is displayed, it is possible to grasp the tissue motion more intuitively than in the case of observation of a moving image. This is useful for a diagnosis of a local area of interest such as a blood clot. Furthermore, in cases where a curve is set by extracting a continuous phase curve, it is possible to support a search for periarterial tissue, in particular, even when it is difficult to identify an area of interest by direction observation in a B mode. This makes it possible to support a doctor in searching for a local area of interest.

The present invention is not limited to the description of the embodiments above, but may be altered by a skilled person within the scope of the claims. An embodiment based on a proper combination of technical means disclosed in different embodiments is encompassed in the technical scope of the present invention.

Further, the image processing device of the present invention is preferably arranged such that the first image generation means includes a first two-dimensional image look-up table that assigns a color gradation value to the two-dimensional image in accordance with a combination of a value of the amplitude of the specific frequency component and a value the phase of the specific frequency component. The foregoing arrangement makes it possible to visually observe the intensity of a periodic tissue motion and spatial continuity in phase of the periodic tissue motion in generating a two-dimensional image.

Further, the image processing device of the present invention is preferably arranged such that the first two-dimensional image look-up table assigns a color gradation value to the two-dimensional image in accordance with the value of the amplitude of the specific frequency component and further assigns a color gradation value to the two-dimensional image in accordance with the value of the phase of the specific frequency component. The foregoing arrangement makes it possible to visually observe the intensity of a periodic tissue motion and spatial continuity in phase of the periodic tissue motion in generating a two-dimensional image.

Further, the image processing device of the present invention is preferably arranged such that the first image generation means includes an amplitude look-up table that assigns a color gradation value to the two-dimensional image in accordance with a value of the amplitude of the specific frequency component and a phase look-up table that assigns a color gradation value to the two-dimensional image in accordance with a value of the phase of the specific frequency component.

The foregoing arrangement makes it possible to, in accordance with amplitude and phase values calculated by the image analysis means, convert these values into color gradation values of a two-dimensional image. This makes it possible to visually observe the intensity of a periodic tissue motion and spatial continuity in phase of the periodic tissue motion in generating a two-dimensional image.

Further, the image processing device of the present invention is preferably arranged such that a two-dimensional image generated in accordance with the amplitude of the specific frequency component and a two-dimensional image generated in accordance with the phase of the specific frequency component are switched between for output. The foregoing arrangement can choose either a two-dimensional image based on the amplitude of a specific frequency component or a two-dimensional image based on the phase of the specific frequency component to be outputted, thereby allowing a user to obtain a desired image.

Further, the image processing device of the present invention is preferably arranged such that the first image generation means repeatedly updates the two-dimensional image generated thereby. The foregoing arrangement makes it possible to display a new two-dimensional image for every update.

Further, the image processing device of the present invention is preferably arranged so as to further include second image generation means for, in accordance with the amplitude of the specific frequency component and the phase of the specific frequency component, generating a two-dimensional image by visualizing a time history of pixel values of a given curve on the cross-section of the specimen.

Further, the image processing device of the present invention is preferably arranged such that, in accordance with the amplitude of the specific frequency component and the phase of the specific frequency component, the second image generation means generates a two-dimensional image by determining a group of pixel values of a given curve on the cross-section of the specimen and visualizes a time history of the group of pixel values.

The foregoing arrangement does not extract a motion from a so-called M-mode echo image, but extracts a motion from a B-mode image. Further, the foregoing arrangement makes it possible to visualize a motion by a given curve.

Further, the image processing device of the present invention is preferably arranged such that the second image generation means includes a second two-dimensional image look-up table that assigns a color gradation value to the two-dimensional image in accordance with a combination of a value of the amplitude of the specific frequency component and a value of the phase of the specific frequency component. The foregoing arrangement makes it possible to visually observe the intensity of a periodic tissue motion and spatial continuity in phase of the periodic tissue motion in generating a two-dimensional image.

Further, the image processing device of the present invention is preferably arranged so as to further include detection means for calculating a given curve by detecting a group of continuous phase coordinates in accordance with the amplitude of the specific frequency component and the phase of the specific frequency component. The foregoing arrangement can detect a continuous phase curve even when it is difficult to identify an area of interest by direct observation in a B mode, thereby facilitating a search for periarterial tissue, for example, in the head of a newborn baby.

Further, the image processing device of the present invention is preferably arranged such that the given curve is externally inputtable. The foregoing arrangement allows a user to input a curve, thereby making it possible to diagnose a local area of interest around a blood clot, for example, in the head of a newborn baby.

Further, the image processing device of the present invention is preferably arranged such that the second image generation means repeatedly updates the two-dimensional image generated thereby. The foregoing arrangement makes it possible to display a new two-dimensional image for every update.

Further, the image processing device of the present invention is preferably arranged such that the frequency analysis means includes Fourier transform means for detecting the amplitude of the specific frequency component and the phase of the specific frequency component according to Fourier transform.

Further, the image processing device of the present invention is preferably arranged such that the specific frequency component is a frequency component equivalent to a heartbeat cycle. According to the foregoing arrangement, the specific frequency component is a frequency component equivalent to a heartbeat cycle, it is possible to find tissue indicative of a periodic motion caused by artery blood flow.

Further, an ultrasonic imaging apparatus of the present invention is preferably arranged so as to include: any one of the above image processing device; and ultrasonic wave transmitting and receiving means for repeatedly receiving an echo signal by scanning a cross-section of a specimen with an ultrasonic beam. The foregoing arrangement can achieve an ultrasonic imaging apparatus that makes it possible to observe a two-dimensional shape of a specimen, the intensity of a periodic motion of the specimen, and spatial continuity in phase of the periodic motion of the specimen.

As described above, an image processing device of the present invention includes: frequency analysis means for detecting amplitude of a specific frequency component and a phase of the specific frequency component by performing a frequency analysis of time-series changes in echo intensity or in Doppler velocity at various points within a cross-section of a specimen, the echo intensity and the Doppler velocity being each obtained by scanning the cross-section of the specimen with an ultrasonic beam; and first image generation means for generating a two-dimensional image based on the amplitude of the specific frequency component and the phase of the specific frequency component thus detected.

As described above, an image processing method of the present invention is an image processing method for use in an ultrasonic imaging apparatus, including the steps of: detecting amplitude of a specific frequency component and a phase of the specific frequency component by performing a frequency analysis of time-series changes in echo intensity or in Doppler velocity at various points within a cross-section of a specimen, the echo intensity and the Doppler velocity being each obtained by scanning the cross-section of the specimen with an ultrasonic beam; and generating a two-dimensional image based on the amplitude of the specific frequency component and the phase of the specific frequency component thus detected.

This makes it possible to observe a two-dimensional shape of a specimen, the intensity of a periodic motion of the specimen, and spatial continuity in phase of the periodic motion of the specimen.

The embodiments and concrete examples of implementation discussed in the foregoing detailed explanation serve solely to illustrate the technical details of the present invention, which should not be narrowly interpreted within the limits of such embodiments and concrete examples, but rather may be applied in many variations within the spirit of the present invention, provided such variations do not exceed the scope of the patent claims set forth below.

### INDUSTRIAL APPLICABILITY

An image processing device of the present invention can be used in a medical field and, in particular, can be suitably used for observation of the head of a newborn baby.

## Claims

1. An image processing device comprising:
frequency analysis means for detecting amplitude of a specific frequency component and a phase of the specific frequency component by performing a frequency analysis of time-series changes in echo intensity or in Doppler velocity at various points within a cross-section of a specimen, the echo intensity and the Doppler velocity being each obtained by scanning the cross-section of the specimen with an ultrasonic beam; and
first image generation means for generating a two-dimensional image based on the amplitude of the specific frequency component and the phase of the specific frequency component thus detected.

2. The image processing device as set forth in claim 1, wherein the first image generation means includes a first two-dimensional image look-up table that assigns a color gradation value to the two-dimensional image in accordance with a combination of a value of the amplitude of the specific frequency component and a value the phase of the specific frequency component.

3. The image processing device as set forth in claim 2, wherein the first two-dimensional image look-up table assigns a color gradation value to the two-dimensional image in accordance with the value of the amplitude of the specific frequency component and further assigns a color gradation value to the two-dimensional image in accordance with the value of the phase of the specific frequency component.

4. The image processing device as set forth in claim 1, wherein the first image generation means includes an amplitude look-up table that assigns a color gradation value to the two-dimensional image in accordance with a value of the amplitude of the specific frequency component and a phase look-up table that assigns a color gradation value to the two-dimensional image in accordance with a value of the phase of the specific frequency component.

5. The image processing device as set forth in claim 4, wherein a two-dimensional image generated in accordance with the amplitude of the specific frequency component and a two-dimensional image generated in accordance with the phase of the specific frequency component are switched between for output.

6. The image processing device as set forth in any one of claims 1 to 5, wherein the first image generation means repeatedly updates the two-dimensional image generated thereby.

7. The image processing device as set forth in claim 1, further comprising second image generation means for, in accordance with the amplitude of the specific frequency component and the phase of the specific frequency component, generating a two-dimensional image by visualizing a time history of pixel values of a given curve on the cross-section of the specimen.

8. The image processing device as set forth in claim 1, further comprising second image generation means for, in accordance with the amplitude of the specific frequency component and the phase of the specific frequency component, generating a two-dimensional image by determining a group of pixel values of a given curve on the cross-section of the specimen and visualizing a time history of the group of pixel values.

9. The image processing device as set forth in claim 7 or 8, wherein the second image generation means includes a second two-dimensional image look-up table that assigns a color gradation value to the two-dimensional image in accordance with a combination of a value of the amplitude of the specific frequency component and a value of the phase of the specific frequency component.

10. The image processing device as set forth in any one of claims 7 to 9, further comprising detection means for calculating a given curve by detecting a group of continuous phase coordinates in accordance with the amplitude of the specific frequency component and the phase of the specific frequency component.

11. The image processing device as set forth in any one of claims 7 to 10, wherein the given curve is externally inputtable.

12. The image processing device as set forth in any one of claims 7 to 11, wherein the second image generation means repeatedly updates the two-dimensional image generated thereby.

13. The image processing device as set forth in any one of claims 1 to 12, wherein the frequency analysis means includes Fourier transform means for detecting the amplitude of the specific frequency component and the phase of the specific frequency component according to Fourier transform.

14. The image processing device as set forth in any one of claims 1 to 13, wherein the specific frequency component is a frequency component equivalent to a heartbeat cycle.

15. An ultrasonic imaging apparatus comprising:
an image processing device as set forth in any one of claims 1 to 14; and
ultrasonic wave transmitting and receiving means for repeatedly receiving an echo signal by scanning a cross-section of a specimen with an ultrasonic beam.

16. An image processing method for use in an ultrasonic imaging device, comprising the steps of:
detecting amplitude of a specific frequency component and a phase of the specific frequency component by performing a frequency analysis of time-series changes in echo intensity or in Doppler velocity at various points within a cross-section of a specimen, the echo intensity and the Doppler velocity being each obtained by scanning the cross-section of the specimen with an ultrasonic beam; and
generating a two-dimensional image based on the amplitude of the specific frequency component and the phase of the specific frequency component thus detected.
